(19) Europäisches Patentamt
European Patent Office
Office européen des brevets

(11) Publication number: **0 193 974**
**B1**

# (12) EUROPEAN PATENT SPECIFICATION

(45) Date of publication of patent specification: **23.11.88**

(51) Int. Cl.⁴: **B 01 J 31/16**, C 07 C 5/31

(21) Application number: **86105626.5**

(22) Date of filing: **17.02.84**

(60) Publication number of the earlier application in accordance with Art. 76 EPC: **0 118 801**

## (54) A catalyst for isomerization reaction.

(30) Priority: **09.03.83 JP 39799/83**

(43) Date of publication of application: **10.09.86 Bulletin 86/37**

(45) Publication of the grant of the patent: **23.11.88 Bulletin 88/47**

(84) Designated Contracting States: **CH DE FR GB LI**

(56) References cited:

**THE JOURNAL OF ORGANIC CHEMISTRY, vol. 44, no. 3, 1979, pages 385-391, American Chemical Society, US; R.B. KING et al.: "Polymer-anchored cobalt tetraarylporphyrin catalysts for the conversion of quadricyclane to norbornadiene"**

(73) Proprietor: **SUMITOMO CHEMICAL COMPANY, LIMITED
15 Kitahama 5-chome Higashi-ku
Osaka-shi Osaka 541 (JP)**

(72) Inventor: **Yoshida, Zenichi
281, Ginzacho-4-chome
Fushimi-ku Kyoto (JP)**
Inventor: **Miki, Sadao
Paresutehto-Nissei 306 43-2, Sanohcho
Shogoin Sakyo-ku Kyoto (JP)**

(74) Representative: **Henkel, Feiler, Hänzel & Partner
Möhlstrasse 37
D-8000 München 80 (DE)**

(56) References cited:
**CHEMICAL ABSTRACTS, vol. 89, no. 4, 24th July 1978, page 570, abstract no. 33060k, Columbus, Ohio, US; L. MUELLER: "Effect of an iron deuteroporphyrin monolayer on oxygen reduction at pyrolytic graphite electrodes"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 193 974 B1

**Description**

The present invention relates to a useful fixed catalyst for the isomerization reaction from quadricyclanes to norbornadienes.

The so-called conversion system from light energy to heat energy in which norbornadienes such as norbornadiene and its derivatives are isomerized into the corresponding quadricyclanes by irradiation with light (first isomerization), the resulting quadricyclanes are converted to the original norbornadienes by isomerization in the presence of a catalyst (second isomerization), and heat generated on said second isomerization is taken out, is well known by Japanese Patent Application Kokai (Laid-Open) Nos. 147,577/1982 and 149,251/1982, "Kagaku to Seibutsu", Vol. 19, No. 2, 80—88, and the like.

In order to operate this light energy conversion system with a good efficiency, the followings should be taken into account: In the second isomerization for returning quadricyclanes, as produced by the first isomerization, to the original norbornadienes, that the catalyst used in said reaction is supported on and fixed to a solid carrier inactive to the reaction, is desired also in preventing the reaction system from pollution, and besides it is very important that the reaction yield is high and particularly the reaction rate also is high. In addition to this, this conversion system is generally operated in a closed and circulating system, so that it is necessary that there is little reduction in the catalytic activity, and that repeated use of a good efficiency is possible.

As the fixed catalyst used in this isomerization reaction, there is known a method to support tetraphenylporphyrin Co(II) [hereinafter, abbreviated to TPPCo(II)] on polystyrene [J. Org. Chem., Vol. 44, No. 3, 385—391]. This TPPCo(II)-polystyrene catalyst, however, had a problem that not only the reaction yeild but also the reaction rate are low, and particularly that repeated use of the catalyst causes a marked reduction in the catalytic activity.

It is the object of the invention to provide fixed catalysts which act to accelerate the reaction rate in said isomerization reaction and which have the various defects inherent to the known fixed catalyst improved.

According to the present invention there is provided a fixed catalyst for the isomerization reaction from quadricyclanes to norbornadienes obtained by adsorption-fixing a deuteroporphyrin IX Co(II) complex [hereinafter, abbreviated to DP IX Co(II)] represented by the formula (I),

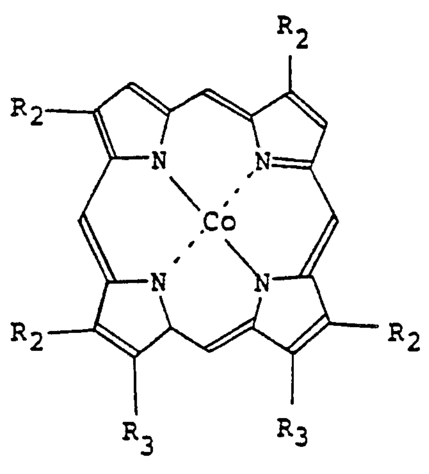

(I)

wherein $R_2$ represents a lower alkyl group and $R_3$ represents a hydroxycarbonyl lower alkyl group, to a modified inorganic carrier in which a polyamine sulfone comprising a recurring unit represented by the formula (II),

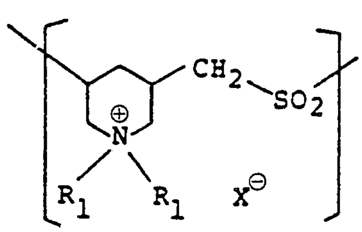

(II)

wherein $R_1$ represents a lower alkyl group and X represents a halogen atom, has been adsorbed on a solid inorganic carrier.

Next, the present invention will be illustrated in detail.

By the term "quadricyclanes", that material used in the isomerization reaction which is an object of the

present invention, are meant various quadricyclane derivatives which include, in addition to quadricyclane having no substituent represented by the formula,

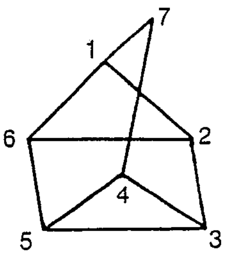

those of which part or all of the 1 to 7 positions have been substituted with one or more members selected from the group consisting of

$-CH_3$, $-COOCH_3$, 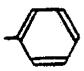, $-CN$, $-CF_3$

In the polyamine sulfone comprising a recurring unit represented by the formula (II), a substituent $R_1$ is a lower alkyl group such as methyl, ethyl, propyl, and a substituent X is a halogen atom such as chlorine, bromine or iodine. In terms of performance, polyamine sulfone in which X is bromine or iodine is more superior to one in which X is chlorine, but chlorine is advantageous economically and practically.

As such polyamine sulfone, those which are already on the market, for example PAS—A® (a product of Nittobo Co.) are usable.

DP IX Co(II) represented by the formula (I) is synthesized, for example, from protohemin. In said formula, $R_2$ is a lower group such as methyl, ethyl, propyl, and $R_3$ is a hydroxycarbonyl lower alkyl group such as hydroxycarbonylmethyl, hydroxycarbonylpropyl, hydroxycarbonylbutyl.

The fixed catalyst of the present invention is obtained by adsorption-fixing DP IX Co(II) to a modified inorganic carrier through an ionic bond, said carrier comprising a solid inorganic carrier having the polyamine sulfone adsorbed thereon. Specifically, the fixed catalyst can be prepared by dipping the modified inorganic carrier in an organic solvent solution, for example pyridine solution of DP IX Co(II) generally at room temperature for 0.5 to 50 hours, and thereafter removing the solvent.

The concentration of DP IX Co(II) in the organic solvent solution varies with the amount of DP IX Co(II) to be supported on the carrier, and it may properly be changed according to the condition of use as catalyst. This amount supported is closely related also to the catalytic activity, and the more the amount, the higher the catalytic activity. Generally, however, the amount of DP IX Co(II) is 0.01 to 50 mg per gram of the modified inorganic carrier.

The modified inorganic carrier used above can be obtained, for example, by dipping an inorganic carrier in an aqueous high-concentration solution, preferably aqueous saturated solution of the polyamine sulfone at room temperature or with heating to impregnate the carrier with the solution, and thereafter, drying the impregnated carrier at 60° to 150°C, preferably 90° to 110°C for 10 to 100 hours.

For applying the fixed catalyst of the present invention to the isomerization reaction from quadricyclanes to norbornadienes, it is carried out by bringing a non-polar solvent solution (e.g. benzene, toluene, pentane, hexane, heptane, chloroform, carbon tetrachloride, carbon disulfide) of quadricyclanes of a proper concentration into contact with the catalyst of the present invention.

In this reaction, the amount of the fixed catalyst used is generally $1 \times 10^{-6}$ to $1 \times 10^{-3}$ mole, as converted to the amount of Co(II) in DP IX Co(II), per mole of quadricyclanes, But, the amount may exceed this range according to reaction conditions such as the form, temperature, etc. of the reaction.

The fixed catalyst of the present invention has very superior characteristics never found in the well-known fixed catalysts as described below: It keeps not only a high conversion of the isomerization reaction, but also a high-level catalytic activity in its repeated use; and besides, its catalytic activity is restored nearly 100% by heat-treatment at 200° to 300°C so that its regeneration and re-use become possible. Consequently, the isomerization reaction can be carried out advantageously by using said catalyst.

Next, the present invention will be illustrated with reference to the following examples.

Catalyst preparation example 1

Polyamine sulfone (PAS—A®, a product of Nittobo Co.) of the formula (II) in which $R_1$ is $CH_3$ and X is Cl, was dissolved in water at room temperature to obtain a saturated aqueous solution. To this solution was added spherical activated alumina (KHA®, produced by Sumitomo Aluminum Refining Co.; particle diameter, 4 to 6 mm) at room temperature and after 15 hours' dipping, the alumina was heat-dried at 100°C for 48 hours to obtain modified alumina.

After this modified alumina was dipped for 24 hours in a thoroughly deaerated pyridine solution of DP IX Co(II) of the formula (I) in which a substituent $R_2$ is $CH_3$ and a substituent $R_3$ is $CH_2CH_2COOH$, the pyridine was removed under reduced pressure to obtain a fixed catalyst having $1.9 \times 10^{-5}$ mole of fixed DP IX Co(II) per gram of the modified alumina.

Example 1

A benzene solution of 2,3-dicyano-1,5,6-trimethylquadricyclane (hereinafter, abbreviated to Q) was brought into contact with a dichloromethane solution of each catalyst shown in Table 1 at 30°C for 12 hours with stirring in a flask to isomerize Q into the corresponding norbornadiene (hereinafter, abbreviated to N). As a result, the rate of isomerization of Q→N with each catalyst was as shown in Table 2.

Hereupon, the amount of each catalyst used was 0.3 time by mole based on Q, and the catalyst of the present invention was used as solid without using a solvent.

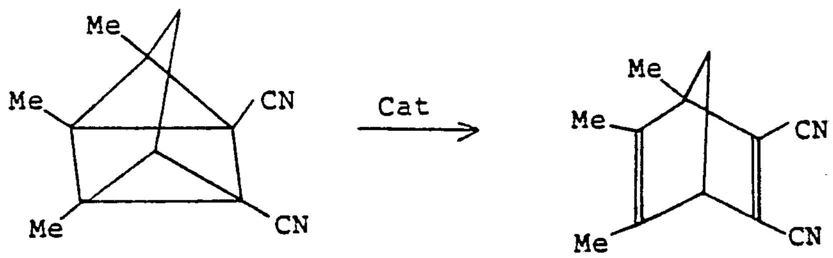


(Q) (N)

Table 1

| | No. | Catalyst | Rate of isomerization of Q → N (%) |
|---|---|---|---|
| Present example | 1 | DP IX Co(II) | 100 |
| Comparative example | 1 | TPP Co(II) (TPP=tetraarylporphyrin) | 18.6 |
| | 2 | 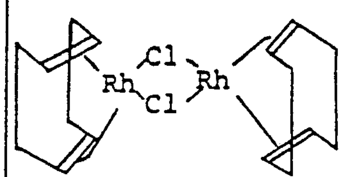 | 3 |
| | 3 | $AgBF_4$ | 52.1 |

Example 2

Ten milliliters of a pentane solution containing 0.1 mole of quadricyclane dissolved was isomerized using $3.1 \times 10^{-6}$ mole, as converted to the amount of Co(II) in DP IX Co(II), of the fixed catalyst prepared in the catalyst preparation example, and a period of time required for 50% of the quadricyclane, a material, to be converted to norbornadiene (half-life period) was measured. As a result, it was found that the half-life period was 18.0 minutes. Further, this isomerization reaction was carried out over and over under the same condition as above except that the catalyst alone was used repeatedly, and the half-life period was measured at each repeated use of the catalyst. As a result, it was found that the half-life period at the 8th repeated use was 32.1 minutes, which means that the catalytic activity at that time is 56% (18.0/32.1 × 100%) of that at the 1st use.

For comparison, the isomerization reaction was repeated in completely the same manner as above using TPPCo(II) supported on polystyrene as a catalyst. As a result, it was found that the half-life period at the 1st use of the catalyst was 44.6 minutes and that at the 6th repeated use was as very long as 88.2 minutes. This means that the catalytic activities at the 1st use and 6th repeated use in this comparative example are 40% and only 20%, respectively, of the catalytic activity at the 1st use, as taken as a standard, of the catalyst of the present invention.

The above test results are shown in Table 2.

Table 2

| | Example | | Comparative example | |
|---|---|---|---|---|
| Number of repetitions | Catalyst: DP IX Co(II)-activated alumina | | Catalyst: TPPCo(II)-polystyrene | |
| | Catalytic activity | | Catalytic activity | |
| | Half-life period (min) | Activity ratio | Half-life period (min) | Activity ratio |
| 1 | 18.0 | 1 | 44.6 | 0.40 |
| 2 | 19.4 | 0.98 | 62.8 | 0.29 |
| 3 | 20.1 | 0.89 | 80.2 | 0.22 |
| 4 | 26.2 | 0.68 | 89.6 | 0.20 |
| 5 | 25.1 | 0.72 | 83.1 | 0.22 |
| 6 | 26.5 | 0.68 | 88.2 | 0.20 |
| 7 | 31.2 | 0.58 | | |
| 8 | 32.1 | 0.56 | | |

In this example, on treating the fixed catalyst, as used eight times over, at 200°C for 10 hours under reduced pressure, the catalyst recovered almost the same performance as that at the 1st use. While in the comparative example, the catalyst used six times over showed no recovery effect even by the same treatment.

**Claim**

A fixed catalyst for the isomerization reaction from quadricyclanes to norbornadienes obtained by adsorption-fixing a deuteroporphyrin IX Co(II) complex represented by the formula,

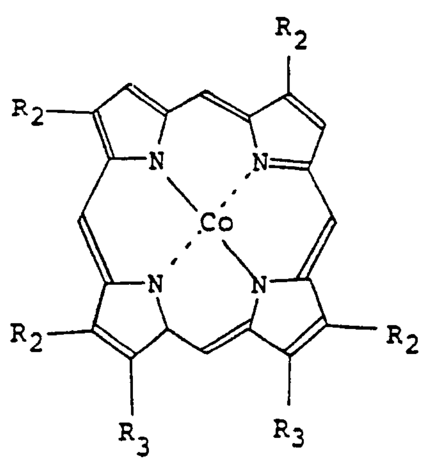

(I)

wherein $R_2$ represents a lower alkyl group and $R_3$ represents a hydroxycarbonyl lower alkyl group, to a modified inorganic carrier in which a polyamine sulfone comprising a recurring unit represented by the formula,

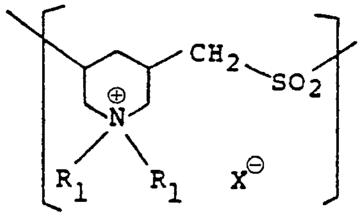

(II)

wherein $R_1$ represents a lower alkyl group and X represents a halogen atom, has been adsorbed on a solid inorganic carrier.

**Patentanspruch**

Fixierter Katalysator für die Isomerisierungsreaktion von Quadricyclanen zu Norbornadienen, erhalten durch adsorptives Fixieren eines Deuteroporphyrin IX Co(II)-Komplexes der Formel:

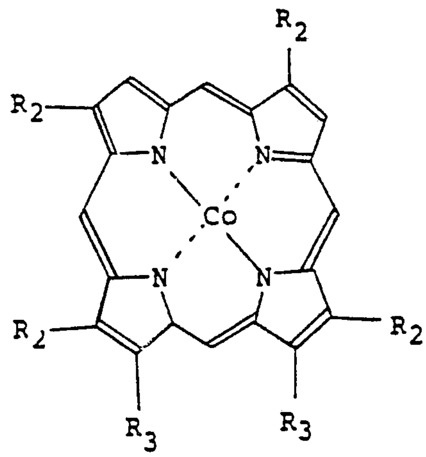

(I)

worin $R_2$ für eine Niedrigalkylgruppe steht und $R_3$ eine Hydroxycarbonylniedrigalkylgruppe darstellt, an einen modifizierten anorganischen Träger, bei dem ein Polyaminsulfon mit einer wiederkehrenden Einheit der Formel:

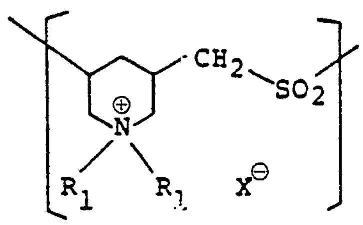

(II)

worin $R_1$ für eine Niedrigalkylgruppe steht und X ein Halogenatom darstellt, an einen festen anorganischen Träger adsorbiert ist.

**Revendication**

Catalyseur fixé sur support pour la réaction de transformation par isomérisation de quadricyclanes en norbornadiënes, obtenu par fixation par adsorption d'un complexe de deutéroporphyrine IX-Co(II) représenté par la formule (I)

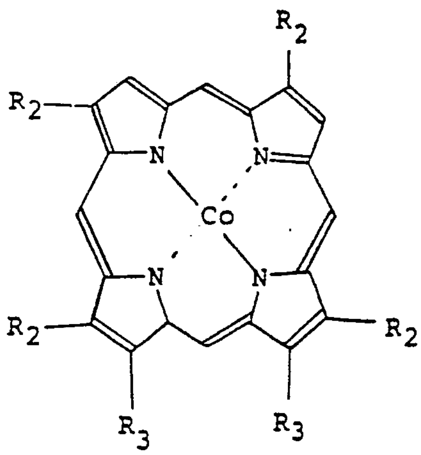

(I)

dans laquelle $R_2$ représente un groupement alkyle inférieur et $R_3$ représente un groupement alkyle inférieur hydroxycarbonylé, sur un support inorganique modifié dans lequel une polyamine-sulfone, comprenant une unité structurale répétitive représentée par la formule (II)

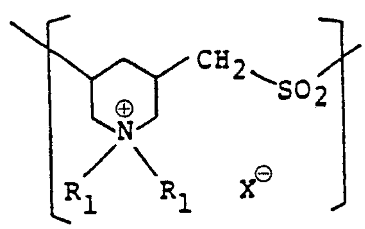

(II)

dans laquelle $R_1$ représente un groupement alkyle inférieur et X représente un atome d'halogène, a été adsorbée sur un support inorganique solide.